(19) Europäisches Patentamt
European Patent Office
Office européen des brevets

(11) Publication number: 0 424 164 B1

# EUROPEAN PATENT SPECIFICATION

(45) Date of publication of patent specification: 06.09.95

(51) Int. Cl.⁶: **B29C 44/46**, B32B 31/06

(21) Application number: 90311474.2

(22) Date of filing: 18.10.90

(54) Process and apparatus for preparing a polymer-based foam.

(30) Priority: 18.10.89 US 422954

(43) Date of publication of application:
24.04.91 Bulletin 91/17

(45) Publication of the grant of the patent:
06.09.95 Bulletin 95/36

(84) Designated Contracting States:
AT BE CH DE DK ES FR GB GR IT LI LU NL SE

(56) References cited:
EP-A- 0 032 624        EP-A- 0 335 669
FR-A- 1 547 335        GB-A- 1 034 595
US-A- 2 956 310        US-A- 3 047 449
US-A- 3 804 931        US-A- 3 903 232
US-A- 3 959 049        US-A- 4 267 134

WORLD PATENTS INDEX LATEST Week 8801,
Derwent Publications Ltd., London, GB; AN
88-002566 & JP-A-62 227 354 (FA CARL
FREUDENBERG) 6 October 1987

(73) Proprietor: FERRIS MFG., CORP.
300 West 83rd Street
Burr Ridge
Illinois 60521 (US)

(72) Inventor: Carr, Roy D.
8255 Steepleside
Burr Ridge,
Illinois 60521 (US)
Inventor: Sessions, Robert W.
German Church Road
Hinsdale,
Illinois 60521 (US)
Inventor: Morin, Peter E.
110th Avenue & 143rd Street
Orland Park,
Illinois 60462 (US)

(74) Representative: Knott, Stephen Gilbert et al
MATHISEN, MACARA & CO.
The Coach House
6-8 Swakeleys Road
Ickenham
Uxbridge
Middlesex UB10 8BZ (GB)

## Description

The present invention relates to a process and apparatus for the production of foam products and, more particularly, a process and apparatus for preparing a polymer-based foam of a predetermined thickness that is produced without the need to slice a larger foam bun to the desired thickness. The resulting foam is suitable for use optimally as a surgical or medical dressing.

Historically, the treatment of wounds involved the application of sterile dressings such as gauze and lint to the injured area, both with and without the use of an antiseptic or curative substance. These dressing materials, however, suffer from a disadvantage in that they adhere to the surface being treated or leave fibers in the wound thereby prolonging the recovery period. In an attempt to circumvent the difficulties associated with the use of these materials, recent research and development efforts have yielded new products which are centered around the use of synthetic materials as wound dressings.

The processes by which these synthetic materials, such as films and sponges, are manufactured, vary according to the composition of the synthetic material and the properties desired in the final product. Nevertheless these processes possess many similarities. Generally, each process involves formulating a polymer-based foam precursor, providing a substrate, applying this foam precursor onto the substrate, and curing the resulting foam by applying heat. Such processes are typically extended by slicing a relatively thick foam bun into a final product having the desired thickness. However, the combination of the use of heat to effect curing as well as slicing the resulting product to the desired thickness increase the cost of the final product. Moreover, in the latter case, the addition of an extra step during production significantly lowers the overall efficiency of the process.

The determination of the amount of heat to effect curing of the foam material is further complicated by the desire to retain a certain amount of moisture in the final foam product. This moisture is preferred as it acts to enhance the appearance, texture, and wettability of the product. Thus, the use of heat to effect curing necessitates a complex analysis to determine the optimal amount of heat to use during the curing phase which will effect the proper curing of the foam while not resulting in the final product having less than the desired degree of moisture. Therefore, it would be advantageous to provide a process which would enable a manufacturer to easily produce a polymer-based foam product having a predetermined degree of moisture without necessitating the performance of a complex curing versus moisture loss analysis.

U.S. Patent No. 4,660,553 illustrates a method for making a medical dressing. This process comprises applying a layer of a foamable silicone elastomer onto a substrate which is an absorbent for the composition. A scraper blade or the like is then used to distribute a critical amount of the elastomer onto the substrate. Reinforcing material is subsequently placed into the elastomeric composition and the reinforced foam is allowed to finally cure. In order to effect curing, the foam may be subjected either to ambient temperatures or to heat, the heat being applied to hasten or increase the degree of cure. After the foam has finally cured, the substrate is cut away from the foam product to produce a flexible, reinforced elastomeric foam sheet. The manner in which the elastomer is applied onto the substrate is the only means by which the thickness of the final foam product may be controlled in this process.

U.S. Patent No. 3,959,049 discloses a process for making air permeable artificial leathers. This process comprises incorporating a catalyst, foam stabilizer, and optionally a pigment, into an isocyanate-terminated polyurethane prepolymer thereby forming a solvent free "paint". After the "paint" is formed, it is coated onto release paper and a substrate is applied over the exposed portion of the "paint". Subsequently, the foaming and polymerization reactions proceed in an atmosphere having a dry-bulb temperature between 40°C to 95°C and a relative humidity of at least 60 percent. The presence of heat and water at this stage of the process causes the reaction to advance. During the course of this reaction, the assemblage is passed through rollers which compress the assemblage. After the compression is completed, Examples 1-4 show the assemblage being placed in a dryer at 130°C for three minutes to further advance the foaming and polymerization reactions. The release paper is removed subsequent to this drying procedure.

EP-A-0 032 624 discloses a process for manufacturing a foam board which is said to possess improved evenness. The process comprises placing a plastic foam-forming mixture between upper and lower paper sheets, conveying the laminate structure through metering rollers, and allowing the foam-forming mixture to expand under a constant pressure supplied by a drag platen until the mixture has fully expanded.

Despite the variety of methods for the production of synthetic foams, there remains a need for a process for manufacturing a foam in which the thickness of the resulting foam may be accurately controlled. Further, this thickness should optimally be attainable without requiring a final slicing procedure. Moreover, it would also be advantageous if the process were more energy efficient, with no heating of the foam being required to effect the curing of the foam product.

According to a first aspect of the present invention there is provided a continuous process for the preparation of a polymer-based foam sheet in which the foam is produced from a reaction product capable of curing at ambient temperature formed by the reaction of a reactant composition comprising a prepolymer and water, the preparation of said foam sheet including the steps of providing a continuously moving substrate and curing the polymer-based foam sheet, said process characterised in that the polymer-based foam sheet produced by said process is a porous cellular foam sheet of a predetermined thickness, the reaction product being deposited on the substrate at a rate and in an amount such that the thickness of the sheet formed by the reaction product, if allowed to rise to its fullest extent without undergoing compression, would be greater than said predetermined thickness, allowing said reaction product to begin rising to form a rising foam sheet, passing said rising foam sheet into and through a compression zone, compressing said rising foam sheet to a predetermined degree, removing the rising foam sheet from the compression zone, allowing the rising foam sheet exiting from said compression zone to rise to provide a porous cellular foam sheet of predetermined thickness prior to finally curing said porous cullular foam sheet.

According to a second aspect of the present invention there is provided an apparatus for the continuous preparation of a polymer-based foam sheet during which the foam is produced from a reaction product capable of curing at ambient temperature formed by the reaction of a reactant composition comprising a prepolymer and water, the apparatus including means for reacting said reactant composition and means for providing a continuously moving substrate, the apparatus being characterised in that said foam sheet is a porous cellular foam sheet of predetermined thickness, the apparatus further comprising means suitable for depositing said reaction product onto the substrate at a rate and in an amount such that the thickness of the sheet formed by the reaction product, if allowed to rise to its fullest extent without undergoing compression, would be greater than said predetermined thickness, and compression means for compressing by a predetermined degree a rising foam sheet formed from said reaction product, the substrate conveying the reaction product to said compression means such that the reaction product begins to rise on said substrate forming said rising foam sheet before encountering the compression means, the apparatus being such that on leaving the compression means the rising foam sheet is allowed to rise to provide a porous cellular foam sheet prior to final curing thereof, said reaction product, said rising foam sheet and said porous cellular foam sheet being processed at ambient temperature after having been deposited onto said substrate at least until said polymer based porous cellular foam sheet is finally cured.

The present invention comprises a method and apparatus for the production of a foam sheet having a predetermined thickness. Further, it is contemplated that this sheet may be produced by curing the foam not at elevated temperatures, but at ambient temperature.

In view of the foregoing objects and requirements, it is therefore desirable to select a foam prepolymer that is capable of curing at ambient temperature. Moreover, the prepolymers chosen should also be capable of foaming in an aqueous system in the absence of a catalyst; however, they should not dissolve in the aqueous liquid. Additionally, it is highly desirable that these prepolymers cure to form a porous cellular foam matrix, this matrix enabling both the absorption of external fluids and carriage of the chosen adjuvant by the resulting foam composition. The formation of this cellular foam matrix is preferred due to the large volume that is available not only for absorption, but also to contain the chosen adjuvant.

Prepolymers contemplated by the present invention are preferably used for the production of a medical or surgical dressing, thus they must also be safe for use in the human body. Generally, polyurethane prepolymers are suitable, and optimally, an isocyanate-capped prepolymer is used.

Isocyanate-capped polyether prepolymers, such as those disclosed in U.S. Patent Nos. 3,903,232 and 4,137,200, are suitable for use in the present invention. Such prepolymers should preferably have a defined average isocyanate functionality greater than 2. These prepolymers may be capped with aromatic isocyanates, such as, for example, toluene diisocyanate or methylene diphenyl isocyanate, or with aliphatic isocyanates, such as isophorone diisocyanate. Specific isocyanate-capped polyether prepolymers which have been found suitable for use in the practice of the present invention include prepolymers sold under the trademark HYPOL (W.R. Grace & Co., Lexington, Massachusetts). Examples include HYPOL FHP 2000, HYPOL FHP 2002, HYPOL FHP 3000, HYPOL FHP 4000, HYPOL FHP 5000, HYPOL X6100, and HYPOL hydrogel.

HYPOL 2000, HYPOL 2002, and HYPOL 3000 prepolymers are derived from toluene diisocyanate. FHP 2000 and FHP 2002 both have an equivalent weight (per NCO) of 625, an NCO content of 1.60 meq/g and a specific gravity of 1.19. The viscosity of FHP 2000 is 18,500 cps (Brookfield LVF, #4 Spindle, 12 rpm at 25°C) and that of FHP 2002 is 20,000. FHP 3000 has an equivalent weight (per NCO) of 425, an NCO content of 2.35 meq/g, a specific gravity of 1.15 and a viscosity (measured as described above) of 10,500. HYPOL hydrogel is likewise derived from toluene diisocyanate. It has an NCO content of 0.5-0.9 meq/g and a viscosity of 10,000 to 12,000 cps at 25°C.

Another example of an isocyanate-capped prepolymer suitable for use in the present invention is AQUAPOL prepolymer, commercially available from Freeman Chemical Corporation. AQUAPOL prepolymers, which are derived from toulene diisocyanate, have an NCO-value of 2.5 to 3.0 and are formed from the reaction of toluene diisocyanate and an organic polyether polyol containing at least 40 percent by weight ethylene oxide adducts as described at Col. 2, lines 3-22 of U.S. Patent No. 4,517,326.

A further example of an isocyanate-capped prepolymer suitable for use in the present invention and which is derived from toluene diisocyanate is sold under the trademark TREPOL. This prepolymer is commercially available from Twin Rivers Engineering. TREPOL prepolymers have an NCO content of 1.4 meq/g and a viscosity at 90°C of 4,700 cps.

The HYPOL FHP 4000 and HYPOL FHP 5000 prepolymers are derived from methylene diphenyl diisocyanate. FHP 4000 has an equivalent weight (per NCO) of 476, an NCO content of 2.10 meq/g, a Brookfield viscosity (LVF, #4 Spindle, 12 rpm. at 25°C) of 20,000 and specific gravity of 1.17. FHP 5000 has an equivalent weight (per NCO) of 392, an NCO content of 2.55 meq/g, a Brookfield viscosity (measured as for FHP 4000) of 18,000 and a specific gravity of 1.17.

Another example of an isocyanate-capped prepolymer suitable is HYPOL X6100. This prepolymer is derived from isophorone diisocyanate and has an NCO content of 1.8 meq/g and a viscosity at 25°C of 12,000 cps.

The amount of prepolymer in the reactant composition used to prepare the hydrophilic foam composition is not particularly critical, but depends on a number of factors as will be described in greater detail hereinafter. One factor is the proportion of other components in the reactant composition. However, there should be sufficient prepolymer to form a polyurethane foam, to releasably contain an adjuvant, if desired, and to adequately contain hydrophilic agent. To that end, the ratio of prepolymer to hydrophilic agent should be such that the reactant composition does not degrade or break-up into its separate constituents. Furthermore, while there should be sufficient prepolymer to provide integrity to the foam, there should not be so much prepolymer that the resulting polyurethane composition becomes unworkable. Where the final composition is to be applied to the skin, the resulting foam composition is desirably relatively smooth and soft while exhibiting the desired absorbance characteristics so that it does not irritate or otherwise harm the skin.

The concentration of prepolymer further depends on its isocyanate functionality and the degree of crosslinking desired in the final foam product. In general, the greater the isocyanate functionality, the greater the degree of cross-linking in the cured foam product. Typically, the reactant composition will comprise from about 20% to about 60% by weight prepolymer. Preferably the reactant composition will comprise from about 45% to about 50% by weight of the prepolymer. Advantageously, the prepolymers may be used alone or in combination.

The reactant composition may, if desired, further include a hydrophilic agent which is incorporated into the foam composition to absorb external liquid, such as wound exudate, and to retain such liquid in the composition. When applied to a wound, the hydrophilic agent is believed to work in conjunction with the foam to hold moisture at the surface of the wound. This allows healing agents exuded by the foam to be concentrated and held at the wound surface. At the same time, the hydrophilic agent incorporated into the foam composition is believed to absorb fluid from the wound to assist thickening of the blood, i.e., it serves as a hemostat. Absorption of exudate by the hydrophilic agent, and the subsequent swelling of the agent results in the removal of inflammatory exudates and particles that would otherwise hinder tissue repair or cause eschar formation. Necrotic debris and bacteria are likewise removed as autolysis, i.e. chemical debridement, is stimulated.

The hydrophilic agent is preferably a highly absorbent polymer, commonly known as a superabsorbent polymer. One measure of polymer absorbency is its fluid uptake capability, well known by those skilled in the art. Hydrophilic agents suitable for use in the present invention include polymers that are capable of absorbing at least fifty times their weight of water, that is, such agents have a fluid uptake of at least 50 ml/g. Hydrophilic agents having an even higher fluid uptake, such as of at least about 100 ml/g and even higher are also acceptable, with those having an uptake of at least about 150 ml/g being preferred. Suitable superabsorbent polymers include sodium and aluminum salts of starch, grafted copolymers of acrylates and acrylamides, and combinations thereof, as well as polyacrylate salts. Of course, other absorbent materials may be used in combination with such highly absorbent polymers, provided the fluid uptake of the overall combination used for the hydrophilic agent is greater than 50 ml/g. When such agents are employed, either alone or in combination, the resulting foam composition desirably has the ability to hold at least about three times its weight in liquid. In the preferred embodiment, the resulting foam composition will have the ability to tightly hold at least about three times its weight in fluid. As used herein "tightly held" or "tightly bound" liquid means the relative amount of liquid retained by the sample after compression. More specifically, retained liquid is the unit weight of liquid per unit weight of foam. It is determined by the formula:

$$\text{Retained Liquid} = \frac{(\text{Wt. of Foam Sample Plus Absorbed Liquid})\ -\ (\text{Weight of Foam Sample})}{\text{Weight of Foam Sample}}$$

Tightly held liquid is a measure of the relative amount of liquid retained by the sample after compression. It is determined by rolling an 8 pound roller over the sample ten times, and then, using the retained liquid formula to calculate the relative proportion of liquid that was not squeezed out of the composition.

Hydrophilic polymers which have been found suitable for use in the foam composition of this invention are commercially available from Grain Processing Corporation. These polymers include a starch-g-poly(2-propenamide-co-2-propenoic acid, mixed sodium and aluminum salt) sold under the trademark WATER LOCK A-222; a starch-graft copolymer of polyacrylic acid and polyacrylamide having the chemical name starch-g-poly(2-propenamide-co-2-propenoic acid, sodium salt), sold under the trademark WATER LOCK A-100; a starch g-poly(2-propenamide-co-2-propenoic acid, sodium salt), sold under the trademark WATER LOCK A-200. Superabsorbent polymers commercially available from Grain Processing Corporation under the trademark WATER LOCK D-212 and WATER LOCK D-242 are likewise suitable. These polymers have the chemical name starch-g-poly(2-propenamide-co-2-propenoic acid, mixed sodium and aluminum salt). The superabsorbent polymer commercially available under the trademark WATER LOCK G-400 is also suitable for use in the making of the hydrophilic foam composition of the present invention. This superabsorbent polymer may be chemically identified as a poly(2-propenamide-co-2-propenoic acid, sodium salt). Other super absorbent powders suitable for use in the present invention are sold by Grain Processing Corporation under the trademark WATER LOCK B, C, and H.

Another example of a suitable superabsorbent polymer is poly-2-propenoic acid, sodium salt, sold under the trademark AQUA KEEP J-500 supplied by Sanyo Corp. In addition, super-absorbent polymers sold by Arakawa Chemical (USA) Inc. under the trademark ARASORB are also suitable. The preferred hydrophilic polymers are WATER LOCK A-100, A-200, A-222 and AQUA KEEP J-500. The hydrophilic polymers may be used alone, or in combination, to achieve the desired absorptivity characteristics in the foam composition.

The hydrophilic agent may comprise additives in addition to the superabsorbent polymers, provided, as discussed above, the additives do not reduce the fluid uptake of the hydrophilic agent to below about 50 ml water per gram of hydrophilic agent and the fluid uptake of the final foam composition is not less than about 3 times its weight. Examples of such additives include methylcellulose, guar gum, pectin, karaya gum, chitosan, agar, acacia powder, carrageenan, gelatin, and combinations thereof.

The amount of hydrophilic agent used and the type of agent, in terms of its fluid uptake, that may be satisfactorily used to make the foam composition is not critical, but is, instead, dependent on the intended application of the resulting foam composition. Stated another way, the greater the quantity of external liquid to be absorbed, e.g., the greater the amount of wound exudate, the greater the amount of hydrophilic agent that should be employed. In the alternative, the greater the amount of wound exudate to be absorbed, the greater the fluid uptake of the hydrophilic agent should be. For example, for an ulcerated wound where there is a high volume of wound exudate, a hydrophilic agent with high uptake is desirable. In addition, it may well be determined that the amount of hydrophilic agent may need to be increased. On the other hand, where the foam is to be applied to a small cut or light burn, it may be suitable to use less hydrophilic agent or to use a hydrophilic agent with a lower fluid uptake. Determination of the type and amount of hydrophilic agent used is well within the ability of one skilled in the art in light of the disclosure herein.

The amount of hydrophilic agent utilized should not be so great as to undesirably reduce the strength of the foam composition or result in a loss of polymer from the foam, although some loss of hydrophilic agent may be tolerated without adversely affecting the ability of the foam to absorb external liquids. The amount of hydrophilic agent employed in the reactant composition will also depend on the absorbency of the material used. As previously indicated, it is preferable that a sufficient amount of hydrophilic agent be employed so that the resulting foam composition is capable of absorbing at least about three times its weight in external liquid. Typically this can be achieved by including from about 5 wt.% to about 20 wt.% hydrophilic agent in the reactant composition.

The reactant composition of this invention may further include an adjuvant; preferably, a water-soluble adjuvant. The adjuvant is releasably carried by the resulting foam composition for subsequent release to a chosen situs of application. Release of the adjuvant occurs in the presence of an external liquid, such as wound exudate, which is preferentially absorbed by the foam composition. Absorption of the external liquid causes at least a portion of the adjuvant to be released.

It will be appreciated by those skilled in the art that not all of the liquid adjuvant is necessarily released (or need it be) in the presence of the external fluid. However, a sufficient amount of adjuvant must be released in order to achieve the desired result. To that end, it will be appreciated that the efficacy of the adjuvant is realized upon its release from the foam composition to the situs of application. In the case of a wound dressing, the situs is the wound, burn or the like, itself. Release of the adjuvant thus provides beneficial treatment to the wound.

Prior to curing, the adjuvant serves as a plasticizer for the reactant composition. It extends the curing time of the composition thereby allowing it to be more thoroughly mixed and formed. Once cured, the foam composition is softened by the adjuvant, allowing the foam to be more pliable and more easily applied to the skin surface or other surface of choice. Additionally, the adjuvant may be somewhat hygroscopic lending further to the hydrophilic nature of the foam composition.

Adjuvants suitable for use in the foam composition of the present invention are mono, di and polyhydric alcohols. Preferably the adjuvants are water soluble so that they may be readily released from the composition upon contact of the foam composition with an external liquid. For wound dressing applications, it is also desirable that the adjuvant be capable of contacting skin without adverse side effects. To that end, it is also preferable that the adjuvant comprise a chemical compound that will have the ability to open the skin pores to achieve a demulcent effect to relieve pain and/or irritation and to achieve an emollient effect to soften the skin and prevent maceration. It is also preferred that the adjuvant be compatible with therapeutic or other agents which may be carried by the adjuvant for subsequent delivery to the situs of application. Suitable adjuvants include water soluble alcohols, including monols, diols and polyhydric alcohols. Examples of monols include ethyl alcohol and isopropyl alcohol. Exemplary of suitable diols are propylene glycol, polyethylene glycol, and polypropylene glycol. Exemplary of suitable polyhydric alcohols are glycerin, 1,2,4-butanetriol, trimethylolpropane, pentaerythritol, and sorbitol. In general, the molecular weight of the alcohols should be less than about 1000. Mixtures of alcohols can likewise be used.

Glycerin is the preferred adjuvant because it has the attributes of a medicament, cosmetic, or therapeutic agent. When glycerin is used and the hydrophilic agent is starch-based, it is believed that glycerin coats the hydrophilic agent to form a starch glycerite. When fluid is absorbed by the foam, glycerin is released, thereby allowing the hydrophilic agent to swell as it absorbs fluid from the wound and causing the foam to conform to the wound contour.

Various additional medicaments, cosmetics, and therapeutic agents may, if desired, be carried with the adjuvant and released with it to the desired situs. This release thus allows the transmission of such therapeutic or other agents carried in the adjuvant to the area of application outside the foam composition, further assisting in the beneficial treatment of the wound.

Illustrative of therapeutic agents which may be incorporated into the foam composition are Collasol 2400, Crotein SPA, Cromoist HYA, Crotein CAA and hydrocortisone acetate. Illustrative of cosmetic agents which may be incorporated into the foam composition are European Collagen Complex, Capture Complex Liposomes, Sardo® bath oil, a hand lotion sold under the trademark Jergens®, Noxema® skin cream, Oil of Olay® BF, Keri® lotion, Vaseline® herbal and aloe lotion, Ben Gay® ointment, and Retin-A® cream.

The amount of adjuvant included in the reactant composition should preferably be sufficient to impart softness and pliability to the foam composition and be capable of delivering a therapeutic agent or the like, if included, to the environment of application. However, the volume of adjuvant should not be so great as to weaken or gel the composition. Generally, it has been found that the amount of adjuvant in the reactant composition should be from about 5 wt.% to about 30 wt.% of the reactant composition.

A wetting agent may be included in the reactant composition to provide more uniform wettability of the resulting foam. The wetting agent also aids in controlling the cell size of the foam and in the reticulation of the final foam. Wetting agents suitable for use include non-ionic surfactants. Examples of materials that may be used as the wetting agent, either alone or in admixture, include block copolymers of ethylene oxide and propylene oxide sold under the trademark PLURONIC by BASF Wyandotte Corporation, ethoxylated sorbitan fatty acid esters, glycerol esters, polyglycerol esters, and silicone fluids. PLURONIC F-68 and L-62 are preferred. As is known, PLURONIC F-68 aids in wound cleansing without causing tissue damage. The use of PLURONIC F-68 is especially preferred because of its cleansing action, particularly because a portion of the surfactant may be released when the foam composition is exposed to the exudate of the wound. Generally, the amount of wetting agent should be from about 1% to about 10% by weight of the reactant composition, preferably from about 5% to about 7% by weight.

The wetting agent should not react with the foam composition or any component of the foam formulation to create difficulties during foam formation or to adversely affect the desired characteristics of the foam composition in use or while being stored.

Water is a necessary component of the reactant composition as its presence results in the initiation of the foaming reaction. It should be appreciated that the source of the water required for the foaming reaction is not critical. The water so required may be provided as a separate component of the reactant composition, or, for example, it may be provided by one of the other components of the reactant composition. By way of illustration, and not in limitation, the required water may be provided with an aqueous-based cosmetic which may be incorporated into the foam composition.

The type of water used is likewise not critical. However, for medical applications, purified water such as deionized or distilled water may be used. Saline solutions may also be used satisfactorily.

It will be appreciated that the relative proportion of prepolymer, adjuvant and hydrophilic agent, if the latter two are included in the reactant composition, can be varied over wide ranges in order to prepare a hydrophilic foam composition having the desired release and exchange characteristics previously described, while likewise providing a foam composition that is aesthetically satisfactory, insofar as its oilyness, touch, appearance and general feel. In general, for use as a wound dressing, it is preferable that the foam composition be soft and generally smooth to the touch so that it does not irritate the skin. These characteristics may be achieved by properly balancing the relative proportion of adjuvant, prepolymer, hydrophilic agent, wetting agent, and water.

By way of illustration, it has been found that if excess glycerin is used in the reactant composition the resulting foam composition has an extended cure time and a decreased ability to tightly hold external liquid. Further, it may have an oily or spongy nonuniform surface. On the other hand, if insufficient glycerin is included in the reactant composition, the resulting foam composition has been found to be less uniform, has relatively poor flow and porosity characteristics, has relatively poor dimensional stability, and absorbs liquid at a slower rate.

Similarly, if the relative proportion of prepolymer to hydrophilic agent is too high or too low, the resulting product will not be satisfactory. The amount of hydrophilic agent must be sufficient to absorb the external liquid and to promote the release of the adjuvant. If the amount of hydrophilic agent is too low, there is insufficient absorption of external liquid. On the other hand, if the amount of hydrophilic agent is too high, then the viscosity of the reactant composition will be too high for appropriate mixing.

In general, in order for the foam composition to have the desired liquid release and exchange characteristics and to provide a foam composition that is soft to the touch and not oily, the weight ratio of prepolymer to hydrophilic agent will desirably be in the range of from about 20:1 to about 20:10 and the ratio of prepolymer to adjuvant will desirably be in the range of from about 20:2 to about 20:30.

It will likewise be appreciated that the wetting agent employed and the amount used may effect the characteristics of the resulting foam composition. It is generally desired that the wetting agent be used in an amount such that the foam is substantially uniform and readily wettable.

Throughout this disclosure, and for purposes of illustration only, the invention will be described in detail by referring to the production of a foam product which is manufactured using an isocyanate-capped prepolymer. However, it should be understood we do not intend to be limited to this single prepolymer or to the specific conditions and components contained in the illustrative example, and any prepolymers which function in the same manner as the illustrative isocyanate-capped prepolymer may be satisfactorily employed.

By way of illustrating the process and apparatus of the present invention, the preparation of a reaction product using the preferred component composition disclosed in US Patent 5,064,653 will be discussed. This composition comprises an isocyanate-capped prepolymer, a hydrophilic agent, water, a wetting agent, and an adjuvant.

Once the prepolymer is selected, the reaction product may be prepared by simply mixing the reactants until they are well blended. This method is preferred when only water and a prepolymer are used to produce the reaction product. However, when additional components are introduced, as in the illustrative example, it is preferable to separately prepare an aqueous phase or phases and an organic phase or phases, with the prepolymer being confined separately. The aqueous phase is prepared by dissolving the hydrophilic agent and wetting agent in water. Heat may be required in order to fully dissolve or disperse the wetting agent. An organic phase or phases may also be prepared if there is an organic component other than the hydrophilic agent. In the illustrative example, the adjuvant is the only other component other than the polymer itself which will comprise the organic phase and, as such, it may be simply contained within a reservoir tank until needed.

7

Although the aqueous phase may be prepared at ambient temperature, it is preferable to maintain the temperature of the aqueous phase at from about 15.6°C (60°F) to about 48.9°C (120°F), particularly when a wetting agent is used. The temperature at which the aqueous phase is most advantageously maintained is about 37.8°C (100°F). This temperature is preferred due to its advantageous effect upon the dispersion of the components in the aqueous phase as well as its effect on the rate of the water-prepolymer reaction.

After each of the aqueous and organic phases are separately prepared, they are combined with the prepolymer and allowed to react and form the reaction product. It should be appreciated that the various blends and phases may be prepared by either a batch process or a continous process.

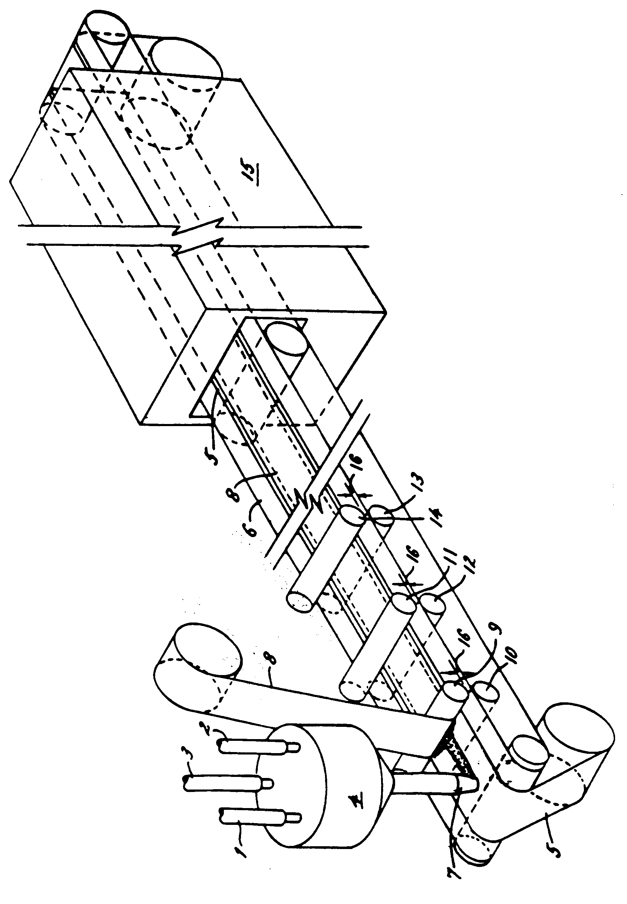

Turning to FIG. 1 which serves to illustrate the present method and apparatus of the present invention, the adjuvant (or organic phase), prepolymer, and aqueous phase are transferred via inlet tubes 1, 2, and 3 to a suitable reaction vessel 4 in which the phases and prepolymer are combined for reaction. The reaction vessel 4 merely serves to mix the reactants sufficiently such that they will react to form the reaction product. The vessel 4 is preferably equipped with speed-controllable mixing paddles to blend the phases and a temperature control means for controlling the temperature of the reactants.

The mixing speed of the vessel 4 and its temperature are preferably set to a predetermined level as a variance in either parameter will affect the properties of the resulting foam sheet. Generally, the predetermined levels are dependent on the flow rates of each component and more specifically on the combined flow rate. For example, if the mixer revolutions per minute (rpm) is too low, inadequate mixing of the reactants results. If the mixer rpm is too high, the heat build up due to the high setting increases the reaction rate of the reactants, thereby effecting the subsequent processing of the reaction product. By way of illustration, if the reaction proceeds too quickly, the reaction product may prematurely cure such that the thickness of the resulting product may not be able to be as accurately controlled by the subsequent compression step. This phenomenon will be explained in more detail hereinafter. Premature failure of parts and excessive use of energy may also occur when the mixing speed is not optimized.

The temperature of the mixer is generally kept lower than the temperature of the reactants because the reaction itself is exothermic. If the temperature is too high, the reaction will proceed at a much higher rate, thereby effecting subsequent processing of the foam, for the reasons stated previously, and also shortening the cure time. Excessive temperatures can also cause an imbalance in carbon dioxide generation and polymerization which may result in a nonuniform product.

The mixing vessel is thus desirably operated at a mixing speed in the range of from about 100 to about 10,000 rpm, and at a temperature within the range of from about 15.6°C (60°F) to about 48.9°C (120°F). Optimally, the aqueous and organic phases are mixed in vessel 4 at a mixing speed of approximately 2500 rpm and at a temperatue of about 32.2°C (90°F).

After the mixing process is completed, the reaction product is discharged from vessel 4 through a nozzle 7 onto a continuously moving substrate 5. The method of and means for depositing the reaction product onto the substrate 5 is not critical to the process of the present invention so long as the reaction product develops a generally uniform thickness prior to being crushed between rollers 9 and 10.

The substrate may be driven by a conveyer belt 6 or, alternatively, the substrate may be drawn in a controlled tension environment such that it will move at the same speed as a conveyor belt would if said belt were used. The controlled tension environment is created by a winder which is located at the end of the production line. As presently contemplated, the turning of this winder will not only wind the finished product into a jellyroll configuration, but will also serve to pull the substrate through the process. Thus, the force exerted by this environment should be sufficient to unwind the substrate from its source and move it through the crushing rollers as well as any additional processing devices that might be present. If this environment is selected, the substrate may either contact or not contact a non-driven conveyor.

In keeping with the present invention, the substrate 5 moves forward relative to the nozzle 7, at the same velocity as the conveyer 6. The flow rate of the reaction product from the nozzle 7 and the velocity of the substrate 5 combined affect the thickness and width of the resulting foam sheet. The velocity of the substrate is directly proportional to the reaction time of the reaction product prior to compression by a first set of compression rollers and effects the nature of the reaction product. For example, the slower the velocity of the substrate, the greater the reaction time (the time between deposition and the initial compression) and the less the width of the final product.

More particularly, the present method and apparatus contemplate that the conveyor 6 be designed so as to allow the velocity of the substrate to vary from about 0.5 to about 55.9 mm/s (about 0.1 to about 11 feet per minute), with the rate at which the reaction product is deposited onto the substrate 5 through nozzle 7 being within the range from about 0.8 to about 15.1 g/s (about 0.1 to about 2.0 pounds per minute). In the preferred embodiment of this invention where the reaction product leaves the reaction vessel at 32.2°C (90°F) after being mixed at 2500 rpm, the reaction product is deposited onto the substrate 5 at a rate of approximately 1.5 g/s (0.2 pounds per minute) and the substrate 5 travels at a velocity of 25.4 mm/s (5.0 feet per minute).

The composition of the substrate material 5 onto which the reaction product is deposited may vary considerably without materially effecting the present process. For example, the substrate may be a porous or non-porous paper or derivative thereof, a type of liner onto which the reaction product will be releasably adhered, a transfer adhesive, or a partially or completely adhesively coated material. However, the material selected must be able to withstand the subsequent processing to which it will be subjected. It is therefore advantageous for the substrate to comprise an adhesive-coated plastic, such as polyurethane or polyvinylidene chloride (saran). The saran should preferably be approximately 1.0 mm (mil) in thickness and be laminated to an approximately 5.5 mm (mil) coated paper for support. Adhesives that are suitable for use will depend upon the end use application of the device. For example, where the device will be used for wound dressings, suitable adhesives include medical grade acrylic adhesives.

The process, as contemplated, is advantageously carried out under a cover or hood. The cover serves to prevent unwanted particulate matter from becoming entrained within the reaction product during processing.

After the reaction product is deposited onto the substrate, but before the reaction product is subjected to its initial compression by rollers 9 and 10, a cover sheet 8 may be deposited onto the product to form a composite. The cover sheet 8, which has previously been release coated, is thus releasably adhered to the product. Advantageously, the cover sheet 8 is a release coated paper or plastic. It is preferred that a polystyrene sheet be used due to its flexibility, availability, and low cost. The polystyrene sheet preferably has one side release coated and is approximately 5.5 mm (mils) in thickness.

The cover sheet 8 may be supplied by any type of suitable device, but preferably the device will provide a continuous supply of cover sheet material. The cover material is positioned onto the surface of the reaction product by a roller. Advantageously the roller which positions the cover sheet may be the same roller that applies the initial compressive force to the reaction product.

Subsequent to, or simultaneously with, the application of the cover sheet 8, the composite is subjected to a compressive force which serves to control the thickness of the resulting foam sheet product. It should be appreciated that the timing of the compression is critical to the success of the process. Specifically, the first compression should not occur until the reaction product has creamed, i.e., until such time as the reaction product begins to foam and rise.

While it is contemplated that the composite may undergo only one compression, it is preferred that it undergo multiple compressions. The compressions are preferably accomplished on a continuous basis by passing the composite through a series of compression means, which in the preferred embodiment comprise a pair of rollers 9, 10 which define a gap 16 therebetween. The compression means compress the creamed foam so as to effect a reduction in foam thickness of from about 5 to about 95 percent of the foam thickness just prior to compression. Reductions of that magnitude may be effected for each of a plurality of compressions. It will be appreciated by those skilled in the art that the number of compressions, degree of compression, and the timing of the compressions is critical to the properties of the final product. These factors will affect the physical properties of the resulting product. More specifically, the density, thickness, width, and appearance of the product will be affected. In order to determine the number and degree of compressions for the particular reaction product and processing conditions employed, a measurement of the foam thickness of the reactant product that has been removed from the conveyor just after each sequential compression should be taken after the foam has been allowed to rise to its fullest extent. This measurement should be compared with a measurement taken of the thickness of the foam reaction product that has similarly been allowed to rise to its fullest extent without undergoing that compression. Such a comparison will allow an operator to determine both the number of compressions and the degree of compression needed to attain a foam having the desired final thickness.

When the compression means comprise at least one pair of spaced apart rollers, which define a gap therebetween through which the composite will pass, as in the illustrative embodiment, the gap between the rollers should be such that the foam will be compressed in the amount of from about 5 percent to about 95 percent, based on the thickness of the foam reaction product as determined by measurements taken of the foam at the times and under the conditions specified in the previous paragraph. For the calculation of the gap between the compression rollers and the placement of the compression rollers from the nozzle, several factors have to be considered. These include the cream and rise times of the reaction product, the percent rise of the reaction product per unit of time, the desired final product characteristics, substrate speed, and the like. These factors should be taken into account when changing conveyor speed, product width or thickness, product formulation, or the like. The rollers should preferably be adjustable to within 0.025 mm (0.001 inch). Optimally these adjustments are made with reference to manually adjustable micrometers which are located on the ends of said rollers. According to the illustrative example, three sets of rollers 9 and 10, 11 and 12, and 13 and 14, are used.

In the illustrative embodiment of the invention, the initial compression preferably reduces the thickness of the reaction product by about 80 percent, and each subsequent compression reduces the thickness by about 40 percent. Compressing the composite in this manner results in a superior final foam product that will emerge having a specific, predetermined thickness. For example, when the velocity of the substrate 5 is 25.4 mm/s (5.0 feet per minute), and the reaction product, which is produced from an isocyanate-capped polyether prepolymer, water, a hydrophilic agent, a wetting agent, and an adjuvant, is deposited at a rate of 1.5 g/s (0.2 pounds per minute), it is preferred that the initial compression, which compresses the foam about 80 percent, occur after the reaction product begins to cream, but no later than 2 seconds after the reaction product leaves the nozzle 7. The second and third compressions, each of which compresses the foam about 40 percent, should also occur within 55 and 70 seconds, respectively after the material has left the nozzle 7.

After the final compression, and after the product no longer adheres to the cover sheet. It is preferred that the cover sheet 8 be removed and the product finally cured. Alternatively, the cover sheet 8 may remain in contact with the reaction product until the reaction product is finally cured, or beyond this time.

An important aspect of the present invention is the production of a foam product without the application of heat to effect the curing process. More specifically, from the time the reaction product leaves the nozzle 7, until the time it is finally cured, the reaction product undergoes processing at ambient temperature. Processing foams of the type contemplated by the present invention at ambient temperature is advantageous in that less energy is consumed and less volatilization of components occurs.

Upon completion of the curing stage of the process, the resulting foam sheet product is at the desired predetermined thickness. No slicing of the foam to achieve the desired thickness is necessary. This results in energy and labor savings to the foam sheet producer, making the present method and apparatus highly desirable when compared with other available methods.

If desired, the foam sheet may subsequently be subjected to drying means wherein the foam sheet is dried to a predetermined moisture level. Preferably the moisture level in the final foam product is 10 percent by weight. The moisture level of the foam is important in that the softness, texture, and wettability of the resulting product are affected by variations in the moisture level. Further, the less moisture in the foam, the more liquids it will absorb and the less problems there will be with warped packaging. Advantageously, drying is carried out using hot air impingement, with the air that is used for the dryer being first drawn through a particulate filter. It is preferable that drying be conducted while the foam is still on the conveyor, but after the cover sheet is removed to enhance drying.

Returning to the illustrative example, the drying temperature should range from about 37.8°C (100°F) to about 79.4°C (175°F) because the components of the product may be volatile or may undergo changes at temperatures higher than 79.4°C (175°F), e.g., the product discolors. Preferably, the temperature is maintained at 60°C (140°F).

The process further contemplates a dryer 15 which is between 0.6 and 6.1 m (2 and 20 feet) in length. The dryer is capable of producing a volume of drying air varying from about 2.8 to 28.3 m$^3$/minute (about 100 to 1000 cubic feet per minute (CFM)). In the preferred embodiment, the product is dried in a 6.1 m (20 foot) dryer at a temperature of 60°C (140°F) with air being supplied at a rate of 14.2 m$^3$/minute (500 CFM), such that the resulting product will have a final moisture content of approximately 10 percent by weight.

The resulting foam sheet product may then be rolled, sterilized, and packaged in an air-impervious container. Alternatively, the sheet may be cut into predetermined lengths or shapes for use in bandages or other wound dressings of various sizes.

Thus, as has been shown, the present invention provides a process for the continuous production of a polymer-based foam in which the thickness of the resulting product may be very accurately controlled and which may be formed directly to a predetermined thickness without the need to slice the foam to the desired thickness after curing. Furthermore, the final product may have a predetermined moisture level and does not require the application of heat to effect curing. Thus the process of the present invention is not only a more energy efficient process, but also one that allows for a higher degree of quality and uniformity of product than was previously thought possible.

While the invention has been described in connection with the preferred embodiment, it is understood that the invention is not intended to be so limited. On the contrary, it is intended to cover all alternatives, modifications, and equivalents as may be included within the scope of the invention as defined by the appended claims. As an example, while the present invention has been described herein as primarily useful for the production of surgical and medical dressings, it should be appreciated that the method is useful for the production of other polymer-based foam products as well.

**Claims**

1. A continuous process for the preparation of a polymer-based foam sheet in which the foam is produced from a reaction product capable of curing at ambient temperature formed by the reaction of a reactant composition comprising a prepolymer and water, the preparation of said foam sheet including the steps of providing a continuously moving substrate (5) and curing the polymer-based foam sheet, said process characterised in that the polymer-based foam sheet produced by said process is a porous cellular foam sheet of a predetermined thickness, the reaction product being deposited on the substrate (5) at a rate and in an amount such that the thickness of the sheet formed by the reaction product, if allowed to rise to its fullest extent without undergoing compression, would be greater than said predetermined thickness, allowing said reaction product to begin rising to form a rising foam sheet, passing said rising foam sheet into and through a compression zone, compressing said rising foam sheet to a predetermined degree, removing the rising foam sheet from the compression zone, allowing the rising foam sheet exiting from said compression zone to rise to provide a porous cellular foam sheet of predetermined thickness prior to finally curing said porous cullular foam sheet.

2. The process of claim 1, wherein the thickness of the rising foam sheet is maintained constant during the compression of the rising foam sheet in the compression zone.

3. The process of claim 1 or claim 2, wherein said prepolymer is a polyurethane prepolymer.

4. The process of claim 3, wherein said polyurethane prepolymer is an isocyanate-capped polyether prepolymer.

5. The process of any preceding claim, wherein said substrate (5) is releasably adherent to said reaction product, said rising foam sheet and said porous cellular foam sheet.

6. The process of any preceding claim, wherein said substrate (5) is selected from the group consisting of polyvinylidene chloride and polyurethane.

7. The process of any preceding claim, further comprising covering said reaction product or said rising foam sheet with a cover sheet (8) after depositing said reaction product onto said substrate (5).

8. The process of claim 7, wherein said cover sheet (8) is releasably adherent to said reaction product and said rising foam sheet.

9. The process of claim 7 or claim 8, wherein said cover sheet (8) is comprised of either paper or plastics.

10. The process of claim 9, wherein said cover sheet (8) is comprised of polystyrene.

11. The process of any of claims 7 to 10, further comprising removing said cover sheet (8) after said porous cellular foam sheet is finally cured.

12. The process of any preceding claim, further comprising drying said porous cellular foam sheet after said porous cellular foam sheet has finally cured.

13. The process of claim 12, wherein said drying is accomplished by dry air impingement.

14. The process of claim 12 or claim 13, wherein said drying is conducted until said porous cellular foam sheet has a moisture content of about 10 percent by weight.

15. The process of any preceding claim, wherein said process is carried out in a particulate-free environment.

16. The process of any preceding claim, wherein said predetermined degree of compression is in the range from about 5 percent to about 95 percent of the thickness of the rising foam sheet just prior to said compression as measured after said rising foam sheet product is allowed to rise to its fullest extent.

17. The process of any preceding claim, wherein said reactant composition further comprises a hydrophilic agent capable of absorbing water.

18. The process of any preceding claim, wherein said reactant composition further comprises an adjuvant in the form of an alcohol.

19. The process of claim 18, wherein said alcohol is selected from the group consisting of monols, diols, and polyhydric alcohols.

20. The process of any preceding claim, wherein said reactant composition further comprises a wetting agent.

21. The process of any preceding claim, wherein said prepolymer is selected from the group consisting of isocyanate-capped polyether polyols having an isocyanate equivalent weight of from about 0.5 meq/g to about 2.5 meq/g and mixtures thereof.

22. The process of any preceding claim, wherein said prepolymer is present in an amount from about 20 wt.% to about 50 wt.% of the total reactant composition.

23. The process of claim 17 or any of claims 18 to 22 when dependent on claim 17, wherein said hydrophilic agent is an absorptive polymer capable of absorbing water and having a fluid uptake of greater than about 50 ml of water per gram of said polymer.

24. The process of claim 17 or any of claims 18 to 23 when dependent on claim 17, wherein said hydrophilic agent is a member selected from the group consisting of starch grafted copolymers of acrylate salts, starch grafted copolymers of acrylamide salts, polyacrylate salts, and mixtures thereof.

25. The process of claim 17 or any of claims 18 to 24 when dependent on claim 17, wherein said hydrophilic agent is present in an amount sufficient to provide a foam composition capable of absorbing at least about 3 times its weight of liquid.

26. The process of claim 25, wherein said foam composition is capable of tightly carrying at least about 3 times its weight of liquid.

27. The process of claim 19 or any of claims 20 to 26 when dependent on claim 19, wherein said alcohol is water soluble.

28. The process of claim 19 or any of claims 20 to 27 when dependent on claim 19, wherein said polyhydric alcohol has a molecular weight of less than about 1000.

29. The process of claim 19 or any of claims 20 to 28 when dependent on claim 19, wherein said alcohol is a member selected from the group consisting of isopropyl alcohol, ethanol, propylene glycol, polyethylene glycol, polypropylene glycol, glycerine, 1,2,4-butanetriol, trimethylolpropane, sorbitol, pentaerythritol, and mixtures thereof.

30. The process of claim 18 or any of claims 19 to 29 when dependent on claim 18, wherein said alcohol is present in an amount from about 5 wt.% to about 30 wt.% of the reactant composition.

31. The process of claim 20 or any of claims 21 to 30 when dependent on claim 20, wherein said wetting agent is a non-ionic surfactant selected from the group consisting of block copolymers of ethylene oxide and propylene oxide, ethoxylated sorbitan fatty acid esters, glycerol esters, polyglycerol esters, silicone fluids and mixtures thereof.

32. The process of claim 20 or any of claims 21 to 31 when dependent on claim 20, wherein said wetting agent is present in an amount from about 1 wt.% to about 10 wt.% of said reactant composition.

33. The process of claim 18 when dependent on claim 17, wherein said prepolymer, said hydrophilic agent and said adjuvant are present in the reactant composition such that the ratio of prepolymer to

12

hydrophilic agent is in the range of from about 20:1 to about 20:10 and the ratio of prepolymer to adjuvant is in the range of from about 20:2 to about 20:30.

34. The process of claim 33, wherein said prepolymer is a member selected from the group consisting of isocyanate-capped polyether polyols having an isocyanate equivalent weight of from about 0.5 meq/g to about 2.5 meq/g and mixtures thereof; said hydrophilic agent is a member selected from the group consisting of starch grafted copolymers of acrylate salts, starch grafted copolymers of acrylamide salts, polyacrylate salts and mixtures thereof; said adjuvant is a member selected from the group consisting of ethanol, isopropyl alcohol, propylene glycol polyethylene glycol, polypropylene glycol, glycerine, 1,2,4-butanetriol, trimethylolpropane, sorbitol, pentaerythritol, and mixtures thereof; said reactant composition includes a wetting agent and said wetting agent is a member selected from the group consisting of block copolymers of ethylene oxide and propylene oxide, ethoxylated sorbitan fatty acid esters, glycerol esters, polyglycerol esters, silicone fluids and mixtures thereof, and said water is a member selected from the group consisting of deionized water, distilled water and normal saline.

35. The process of claim 34, wherein said prepolymer is an isocyanate-capped polyether prepolymer having an isocyanate equivalent weight of about 1.6 meq/g and an equivalent weight per isocyanate group of about 625; said hydrophilic agent is starch-g-poly(2-propenamide-co-2-propenoic acid, mixed sodium and aluminium salt); said adjuvant comprises glycerine, and said wetting agent is a member selected from the group consisting of block copolymers of ethylene oxide and propylene oxide.

36. The process of claim 4 when claim 4 is dependent on claim 1, wherein the continuously moving substrate (5) moves at a rate in the range of between about 0.5 to about 55.9 mm/s (about 0.1 to about 11 feet per minute) and the reaction product is deposited onto the substrate (5) at a rate in the range of about 0.8 to about 15.1 g/s (about 0.1 to about 2.0 pounds per minute).

37. The process of claim 36, wherein said continuously moving substrate (5) moves at a rate of about 25.4 mm/s (5 feet per minute) and said reaction product is deposited onto said substrate (5) at a rate of about 1.5 g/s (0.2 pounds per minute).

38. The process of claim 36 or claim 37, wherein the temperature of said reaction product upon deposition ranges from about 15.6°C (60°F) to about 48.9°C (120°F).

39. The process of any of claims 36 to 38, wherein said predetermined degree of compression is in the range from about 5 percent to about 95 percent of the thickness of the rising foam sheet just prior to said compression as measured after said rising foam sheet is allowed to rise to its fullest extent.

40. The process of any of claims 36 to 39, wherein said compression step comprises subjecting said rising foam sheet to three compressions.

41. The process of claim 40, wherein the degree of the first of said three compressions is about 80 percent of the thickness of the rising foam sheet just prior to the first compression as measured after said sheet is allowed to rise to its fullest extent, the degree of the second compression is about 40 percent of the thickness of the rising foam sheet prior to the second compression as measured after said sheet is allowed to rise to its fullest extent, and the degree of the third compression is about 40 percent of the thickness of the rising foam sheet prior to said third compression as measured after said sheet is allowed to rise to its fullest extent.

42. The process of any of claims 36 to 41, further comprising covering said reaction product or said rising foam sheet with a cover sheet (8) after depositing said reaction product onto said substrate (5).

43. The process of any of claims 36 to 42, wherein said substrate (5) is driven by a continuously moving conveyor belt (6).

44. The process of any of claims 36 to 42, wherein said substrate (5) is drawn by a winding device.

45. The process of claim 42 or either of claims 43 or 44 when dependent on claim 42, further comprising removing said cover sheet (8) after said rising foam sheet is finally compressed and said rising foam

EP 0 424 164 B1

sheet no longer adheres to said cover sheet (8).

46. The process of any of claims 36 to 45, further comprising drying said product after said product is finally cured at a temperature ranging from about 37.8°C (100°F) to about 79.4°C (175°F).

47. The process of claim 46, wherein said drying step is conducted in a particulate-free environment.

48. An apparatus for the continuous preparation of a polymer-based foam sheet during which the foam is produced from a reaction product capable of curing at ambient temperature formed by the reaction of a reactant composition comprising a prepolymer and water, the apparatus including means (1,2,3,4) for reacting said reactant composition and means (6) for providing a continuously moving substrate (5), the apparatus being characterised in that said foam sheet is a porous cellular foam sheet of predetermined thickness, the apparatus further comprising means (7) suitable for depositing said reaction product onto the substrate (5) at a rate and in an amount such that the thickness of the sheet formed by the reaction product, if allowed to rise to its fullest extent without undergoing compression, would be greater than said predetermined thickness, and compression means (9,10,11,12,13,14) for compressing by a predetermined degree a rising foam sheet formed from said reaction product, the substrate (5) conveying the reaction product to said compression means such that the reaction product begins to rise on said substrate forming said rising foam sheet before encountering the compression means, the apparatus being such that on leaving the compression means the rising foam sheet is allowed to rise to provide a porous cellular foam sheet prior to final curing thereof, said reaction product, said rising foam sheet and said porous cellular foam sheet being processed at ambient temperature after having been deposited onto said substrate (5) at least until said polymer based porous cellular foam sheet is finally cured.

49. The apparatus of claim 48, wherein the compression means (9,10,11,12,13,14) is such that the thickness of the rising foam sheet is maintained constant during the compression of the rising foam sheet by said compression means.

50. The apparatus of claim 48 or claim 49, wherein said means for providing a continuously moving substrate includes a conveyor belt (6).

51. The apparatus of claim 48 or claim 49, wherein said means for providing a continuously moving substrate includes a winding device.

52. The apparatus of any of claims 48 to 51, wherein said means for depositing said reaction product comprises a nozzle (7).

53. The apparatus of any of claims 48 to 52, wherein said means for compressing said rising foam sheet comprises at least one pair of rollers (9,10,11,12,13,14).

54. The apparatus of any of claims 48 to 53, wherein said apparatus further comprises means for covering said reaction product or said rising foam sheet sheet with a cover sheet (8) to form a composite.

55. The apparatus of claim 54, wherein said means for covering said reaction product or said rising foam sheet comprises a roller (9).

56. The apparatus of any of claims 48 to 55, further comprising means (15) for drying said polymer-based porous cellular foam sheet after said cellular foam sheet has finally cured.

57. The apparatus of claim 56, wherein said drying means (15) comprises a particulate filter such that said polymer-based porous cellular foam sheet remains substantially particulate-free.

58. The apparatus of any of claims 48 to 57, wherein said apparatus further comprises means for maintaining said reaction product and said rising foam sheet substantially particulate-free.

14

EP 0 424 164 B1

**Patentansprüche**

1. Kontinuierliches Verfahren zur Herstellung einer Schaum-Bahn auf Polymer-Basis, in dem der Schaum aus einem Reaktionsprodukt hergestellt wird, das bei Umgebungstemperatur härten kann und gebildet wird durch Reaktion einer Reaktanden-Zubereitung, die ein Prepolymer und Wasser umfaßt, wobei die Herstellung der Schaum-Bahn die Schritte einschließt, daß man ein sich kontinuierlich fortbewegendes Substrat (5) bereitstellt und die Schaum-Bahn auf Polymer-Basis härtet, wobei das Verfahren dadurch gekennzeichnet ist, daß die Schaum-Bahn auf Polymer-Basis, die durch das Verfahren hergestellt wird, eine poröse Zellschaum-Bahn einer vorbestimmten Dicke ist, wobei das Reaktionsprodukt auf dem Substrat (5) mit einer solchen Geschwindigkeit und in einer solchen Menge abgeschieden wird, daß die Dicke der aus dem Reaktionsprodukt gebildeten Bahn, wenn man sie in vollem Umfang ohne unter Druck zu stehen aufschäumen ließe, größer wäre als die vorbestimmte Dicke, wobei man das Reaktionsprodukt unter Bildung einer aufsteigenden Schaum-Bahn beginnen läßt aufzusteigen, die aufsteigende Schaum-Bahn in eine Kompressionszone einführt und durch diese hindurchführt, die aufsteigende Schaum-Bahn auf einen vorbestimmten Grad zusammendrückt, die aufsteigende Schaum-Bahn aus der Kompressionszone entfernt, die aufsteigende Schaum-Bahn aus der Kompressionszone unter Aufsteigen austreten läßt und so eine poröse Zellschaum-Bahn einer vorbestimmten Dicke schafft, bevor man am Ende die poröse Zell-Schaumbahn härtet.

2. Verfahren nach Anspruch 1, worin die Dicke der aufsteigenden Schaum-Bahn während des Zusammendrückens der aufsteigenden Schaum-Bahn in der Kompressionszone konstant gehalten wird.

3. Verfahren nach Anspruch 1 oder Anspruch 2, worin das Prepolymer ein Polyurethan-Prepolymer ist.

4. Verfahren nach Anspruch 3, worin das Polyurethan-Prepolymer ein mit Isocyanat-Endgruppen versehenes Polyether-Prepolymer ist.

5. Verfahren nach einem der vorangehenden Ansprüche, worin das Substrat (5) an dem Reaktionsprodukt, der aufsteigenden Schaum-Bahn und der porösen Zellschaum-Bahn lösbar haftend ist.

6. Verfahren nach einem der vorangehenden Ansprüche, worin das Substrat (5) aus der aus Polyvinylidenchlorid und Polyurethan bestehenden Gruppe gewählt ist.

7. Verfahren nach einem der vorangehenden Ansprüche, welches außerdem den Schritt umfaßt, daß man das Reaktionsprodukt oder die aufsteigende Schaum-Bahn nach Abscheiden des Reaktionsprodukts auf das Substrat (5) mit einer Abdeck-Bahn (8) abdeckt.

8. Verfahren nach Anspruch 7, worin die Abdeck-Bahn (8) auf dem Reaktionsprodukt und der aufsteigenden Schaum-Bahn lösbar haftet.

9. Verfahren nach Anspruch 7 oder Anspruch 8, worin die Abdeck-Bahn (8) entweder aus Papier oder aus Kunststoff besteht.

10. Verfahren nach Anspruch 9, worin die Abdeck-Bahn (8) aus Polystyrol besteht.

11. Verfahren nach einem der Ansprüche 7 bis 10, welches außerdem den Schritt umfaßt, daß man die Abdeck-Bahn (8) entfernt, nachdem die poröse Zellschaum-Bahn endgehärtet ist.

12. Verfahren nach einem der vorangehenden Ansprüche, welches außerdem den Schritt umfaßt, daß man die poröse Zellschaum-Bahn trocknet, nachdem die poröse Zellschaum-Bahn endgehärtet ist.

13. Verfahren nach Anspruch 12, worin der Vorgang des Trocknens durch Aufblasen von trockener Luft bewirkt wird.

14. Verfahren nach Anspruch 12 oder Anspruch 13, worin der Vorgang des Trocknens durchgeführt wird, bis die poröse Zellschaum-Bahn einen Feuchtigkeitsgehalt von etwa 10 Gew.-% aufweist.

15

**15.** Verfahren nach einem der vorangehenden Ansprüche, worin das Verfahren in einer Teilchen-freien Umgebung durchgeführt wird.

**16.** Verfahren nach einem der vorangehenden Ansprüche, worin der vorbestimmte Kompressionsgrad im Bereich von etwa 5 Prozent bis etwa 95 Prozent der Dicke der aufsteigenden Schaum-Bahn unmittelbar vor dem Kompressionsvorgang liegt, gemessen nachdem man das aufsteigende Schaumbahn-Produkt hat in vollem Ausmaß aufsteigen lassen.

**17.** Verfahren nach einem der vorangehenden Ansprüche, worin die Reaktanden-Zubereitung außerdem ein hydrophiles Mittel umfaßt, das Wasser absorbieren kann.

**18.** Verfahren nach einem der vorangehenden Ansprüche, worin die Reaktanden-Zubereitung außerdem einen Hilfsstoff in Form eines Alkohols umfaßt.

**19.** Verfahren nach Anspruch 18, worin der Alkohol gewählt ist aus der aus Monoolen, Diolen und mehrwertigen Alkoholen bestehenden Gruppe.

**20.** Verfahren nach einem der vorangehenden Ansprüche, worin die Reaktanden-Zubereitung außerdem ein Befeuchtungsmittel umfaßt.

**21.** Verfahren nach einem der vorangehenden Ansprüche, worin das Prepolymer gewählt ist aus der aus mit Isocyanat-Endgruppen versehenen Polyetherpolyolen mit einem Isocyanat-Equivalentgewicht von etwa 0,5 meq/g bis etwa 2,5 meq/g und deren Mischungen bestehenden Gruppe.

**22.** Verfahren nach einem der vorangehenden Ansprüche, worin das Prepolymer in einer Menge von etwa 20 Gew.-% bis etwa 50 Gew.-% der Gesamt-Reaktanden-Zubereitungzugegen ist.

**23.** Verfahren nach Anspruch 17 oder einem der Ansprüche 18 bis 22, wenn sie von Anspruch 17 abhängig sind, worin das hydrophile Mittel ein absorptionsfähiges Polymer ist, das Wasser absorbieren kann und ein Flüssigkeitsaufnahmevermögen größer als etwa 50 ml Wasser pro Gramm Polymer aufweist.

**24.** Verfahren nach Anspruch 17 oder einem der Ansprüche 18 bis 23, wenn diese von Anspruch 17 abhängig sind, worin das hydrophile Mittel eine Verbindung ist, die gewählt ist aus der aus Stärke-gepfropften Copolymeren von Acrylatsalzen, Stärke-gepfropften Copolymeren von Acrylamid-Salzen, Polyacrylat-Salzen und deren Mischungen bestehenden Gruppe.

**25.** Verfahren nach Anspruch 17 oder einem der Ansprüche 18 bis 24, wenn diese von Anspruch 17 abhängig sind, worin das hydrophile Mittel in einer Menge zugegen ist, die ausreichend ist, um eine Schaum-Zubereitung zu schaffen, die wenigstens das 3-fache ihrer Gewichtsmenge an Flüssigkeit absorbieren kann.

**26.** Verfahren nach Anspruch 25, worin die Schaum-Zubereitung wenigstens etwa das 3-fache ihres Gewichts an Flüssigkeit ohne Lecken tragen kann.

**27.** Verfahren nach Anspruch 19 oder einem der Ansprüche 20 bis 26, wenn diese von Anspruch 19 abhängig sind, worin der Alkohol in Wasser löslich ist.

**28.** Verfahren nach Anspruch 19 oder einem der Ansprüche 20 bis 27, wenn diese von Anspruch 19 abhängig sind, worin der mehrwertige Alkohol ein Molekulargewicht von weniger als etwa 1000 aufweist.

**29.** Verfahren nach Anspruch 19 oder einem der Ansprüche 20 bis 28, wenn diese von Anspruch 19 abhängig sind, worin der Alkohol eine Verbindung ist, die gewählt ist aus der aus Isopropylalkohol, Ethanol, Propylenglykol, Polyethylenglykol, Polypropylenglykol, Glycerin, 1,2,4-Butantriol, Trimethylolpropan, Sorbitol, Pentaerythritol und deren Mischungen bestehenden Gruppe.

**30.** Verfahren nach Anspruch 18 oder einem der Ansprüche 19 bis 29, wenn diese abhängig von Anspruch 18 sind, worin der Alkohol in einer Menge von etwa 5 Gew.-% bis etwa 30 Gew.-% der Reaktanden-Zubereitung zugegen ist.

31. Verfahren nach Anspruch 20 oder einem der Ansprüche 21 bis 30, wenn diese von Anspruch 20 abhängig sind, worin das Befeuchtungsmittel ein nicht-ionisches oberflächenaktives Mittel (Tensid) ist, das gewählt ist aus der aus Block-Copolymeren von Ethylenoxid und Propylenoxid, ethoxylierten Sorbitanfettsäureestern, Glycerinestern, Polyglycerinestern, Silicon-Fluiden und deren Mischungen bestehenden Gruppe.

32. Verfahren nach Anspruch 20 oder einem der Ansprüche 21 bis 31, wenn diese von Anspruch 20 abhängig sind, worin das Befeuchtungsmittel in einer Menge von etwa 1 Gew.-% bis etwa 10 Gew.-% der Reaktanden-Zubereitung zugegen ist.

33. Verfahren nach Anspruch 18, wenn dieser von Anspruch 17 abhängig ist, worin das Prepolymer, das hydrophile Mittel und der Hilfsstoff in der Reaktanden-Zubereitung so zugegen sind, daß das Verhältnis des Prepolymers zu dem hydrophilen Mittel im Bereich von etwa 20 : 1 bis etwa 20 : 10 liegt und das Verhältnis des Prepolymers zu dem Hilfsstoff im Bereich von etwa 20 : 2 bis etwa 20 : 30 liegt.

34. Verfahren nach Anspruch 33, worin das Prepolymer eine Verbindung ist, die gewählt ist aus der aus mit Isocyanat-Endgruppen versehenen Polyetherpolyolen mit einem Isocyanat-Equivalentgewicht von etwa 0,5 meq/g bis etwa 2,5 meq/g und deren Mischungen bestehenden Gruppe, das hydrophile Mittel eine Verbindung ist, die gewählt ist aus der aus Stärke-gepfropften Copolymeren von Acrylat-Salzen, Stärke-gepfropften Copolymeren von Acrylamid-Salzen, Polyacrylat-Salzen und deren Mischungen bestehenden Gruppe, der Hilfsstoff eine Verbindung ist, die gewählt ist aus der aus Ethanol, Isopropylalkohol, Propylenglykol, Polyethylenglykol, Polypropylenglykol, Glycerin, 1,2,4-Butantriol, Trimethylolpropan, Sorbitol, Pentaerythritol und deren Mischungen bestehenden Gruppe, die Reaktanden-Zubereitung ein Befeuchtungsmittel einschließt und das Befeuchtungsmittel eine Verbindung ist, die gewählt ist aus der aus Block-Copolymeren von Ethylenoxid und Propylenoxid, ethoxylierten Sorbitanfettsäureestern, Glycerinestern, Polyglycerinestern, Silicon-Fluiden und deren Mischungen bestehenden Gruppe und das Wasser eine Verbindung ist, die gewählt ist aus der aus entionisiertem Wasser, destilliertem Wasser und normaler Kochsalz-Lösung bestehenden Gruppe.

35. Verfahren nach Anspruch 34, worin das Prepolymer ein mit Isocyanat-Endgruppen versehenes Polyether-Prepolymer mit einem Isocyanat-Equivalentgewicht von etwa 1,6 meq/g und einem Equivalentgewicht pro Isocyanat-Gruppe von etwa 625 ist, das hydrophile Mittel Stärke-g-poly-(2-propenamid-co-2-propensäure; Mischung des Natrium- und Aluminium-Salzes) ist, der Hilfsstoff Glycerin umfaßt und das Befeuchtungsmittel eine Verbindung ist, die gewählt ist aus der aus Block-Copolymeren von Ethylenoxid und Propylenoxid bestehenden Gruppe.

36. Verfahren nach Anspruch 4, wenn Anspruch 4 von Anspruch 1 abhängig ist, worin sich das sich kontinuierlich bewegende Substrat (5) mit einer Geschwindigkeit im Bereich zwischen etwa 0,5 und etwa 55,9 mm/s (etwa 0,1 bis etwa 11 Fuß pro Minute) bewegt und das Reaktionsprodukt auf dem Substrat (5) mit einer Geschwindigkeit im Bereich von etwa 0,8 bis etwa 15,1 g/s (etwa 0,1 bis etwa 2,0 pounds pro Minute) abgeschieden wird.

37. Verfahren nach Anspruch 36, worin sich das sich kontinuierlich bewegende Substrat (5) mit einer Geschwindigkeit von etwa 25,4 mm/s (5 Fuß pro Minute) bewegt und das Reaktionsprodukt auf den Substrat (5) mit einer Geschwindigkeit von etwa 1,5 g/s (0,2 pounds pro Minute) abgeschieden wird.

38. Verfahren nach Anspruch 36 oder Anspruch 37, worin die Temperatur des Reaktionsprodukts bei Abscheiden im Bereich von etwa 15,6 °C (60 °F) bis etwa 48,9 °C (120 °F) liegt.

39. Verfahren nach einem der Ansprüche 36 bis 38, worin der vorbestimmte Kompressionsgrad im Bereich von etwa 5% bis etwa 95 % der Dicke der aufsteigenden Schaum-Bahn unmittelbar vor Kompression liegt, gemessen nachdem man die aufsteigende Schaum-Bahn hat in vollem Umfang aufsteigen lassen.

40. Verfahren nach einem der Ansprüche 36 bis 39, worin der Kompressionsschritt den Schritt umfaßt, daß man die aufsteigende Schaum-Bahn drei Kompressionsschritten unterwirft.

**41.** Verfahren nach Anspruch 40, worin der Grad der ersten der drei Kompressionen etwa 80 Prozent der Dicke der aufsteigenden Schaum-Bahn unmittelbar vor dem ersten Kompressionsschritt beträgt, gemessen nachdem man die Bahn hat in vollem Ausmaß aufsteigen lassen, der Grad der zweiten Kompression etwa 40 Prozent der Dicke der aufsteigenden Schaum-Bahn vor der zweiten Kompression beträgt, gemessen nachdem man die Bahn hat in vollem Umfang aufsteigen lassen, und der Grad der dritten Kompression etwa 40 Prozent der Dicke der aufsteigenden Schaum-Bahn vor dem dritten Kompressionsschritt beträgt, gemessen nachdem man die Bahn im vollem Umfang hat aufsteigen lassen.

**42.** Verfahren nach einem der Ansprüche 36 bis 41, welches außerdem den Schritt umfaßt, daß man das Reationsprodukt oder die aufsteigende Schaum-Bahn nach Abscheiden des Reaktionsprodukts auf dem Substrat (5) mit einer Abdeckbahn (8) abdeckt.

**43.** Verfahren nach einem der Ansprüche 36 bis 42, worin das Substrat (5) durch ein sich kontinuierlich bewegendes Förderband (6) angetrieben wird.

**44.** Verfahren nach einem der Ansprüche 36 bis 42, worin das Substrat (5) durch eine Aufwickel-Vorrichtung gezogen wird.

**45.** Verfahren nach Anspruch 42 oder einem der Ansprüche 43 oder 44, wenn diese von Anspruch 42 abhängig sind, welches außerdem den Schritt umfaßt, daß man die Abdeck-Bahn (8) entfernt, nachdem die aufsteigende Schaum-Bahn endkomprimiert wurde und die aufsteigende Schaum-Bahn nicht mehr an der Abdeck-Bahn (8) haftet.

**46.** Verfahren nach einem der Ansprüche 36 bis 45, welches weiter den Schritt umfaßt, daß man das Produkt trocknet, nachdem das Produkt bei einer Temperatur im Bereich von etwa 37,8°C (100°F) bis etwa 79,4°C (175°F) endgehärtet wurde.

**47.** Verfahren nach Anspruch 46, worin der Trocknungsschritt in einer Teilchen-freien Umgebung durchgeführt wird.

**48.** Vorrichtung zur kontinuierlichen Herstellung einer Schaum-Bahn auf Polymer-Basis, während der der Schaum aus einem Reaktionsprodukt hergestellt wird, das bei Umgebungstemperatur härten kann und durch Reaktion einer ein Prepolymer und Wasser umfassenden Reaktanden-Zubereitung gebildet wird, wobei die Vorrichtung Einrichtungen (1,2,3,4) zur Umsetzung der Reaktanden-Zubereitung und Einrichtungen (6) zur Schaffung eines sich kontinuierlich bewegenden Substats (5) einschließt und die Vorrichtung dadurch gekennzeichnet ist, daß die Schaum-Bahn eine poröse Zellschaum-Bahn vorbestimmter Dicke ist und die Vorrichtung außerdem eine Einrichtung (7) umfaßt, die zum Abscheiden des Reaktionsprodukts auf dem Substrat (5) mit einer solchen Geschwindigkeit und in einer solchen Menge geeignet ist, daß die Dicke der aus dem Reaktionsprodukt gebildeten Bahn, wenn man sie in vollem Umfang aufsteigen ließe, ohne daß sie einer Kompression unterworfen wäre, größer wärme als die vorbestimmte Dicke, und Kompressionseinrichtungen (9,10,11,12,13,14) zum Zusammendrücken einer aus dem Reaktionsprodukt gebildeten aufsteigenden Schaum-Bahn um einen vorbestimmten Grad, wobei das Substrat (5) das Reaktionsprodukt zu den Kompressionseinrichtungen in der Weise transportiert, daß das Reaktionsprodukt auf dem Substrat unter Bildung der aufsteigenden Schaum-Bahn aufzusteigen beginnt, bevor es auf die Kompressions-Einrichtungen trifft, wobei die Vorrichtung derart ist, daß man die aufsteigende Schaum-Bahn bei Verlassen der Kompressionseinrichtungen aufsteigen läßt und so eine poröse Zellschaum-Bahn vor deren Endhärtung bereitstellt, wobei das Reaktionsprodukt, die aufsteigende Schaum-Bahn und die poröse Zellschaum-Bahn, nachdem sie auf dem Substrat (5) abgeschieden wurden, wenigstens solange bei Umgebungstemperatur verarbeitet werden, bis die poröse Zellschaum-Bahn auf Polymerbasis endgehärtet wird.

**49.** Vorrichtung nach Anspruch 48, worin die Kompressions-Einrichtung (9,10,11,12,13,14) derart ist, daß die Dicke der aufsteigenden Schaum-Bahn während der Kompression der aufsteigenden Schaum-Bahn durch die Kompressionseinrichtung konstant gehalten wird.

**50.** Vorrichtung nach Anspruch 48 oder Anspruch 49, worin die Einrichtung zum Schaffen eines sich kontinuierlich bewegenden Substrats ein Förderband (6) einschließt.

18

**51.** Vorrichtung nach Anspruch 48 oder Anspruch 49, worin die Einrichtung zum Schaffen eines sich kontinuierlich bewegenden Substrats eine Aufwickel-Vorrichtung einschließt.

**52.** Vorrichtung nach einem der Ansprüche 48 bis 51, worin die Einrichtung zum Abscheiden des Reaktionsprodukts eine Düse (7) einschließt.

**53.** Vorrichtung nach einem der Ansprüche 48 bis 52, worin die Einrichtung zum Zusammendrücken der aufsteigenden Schaum-Bahn wenigstens ein Walzenpaar (9,10,11,12,13,14) umfaßt.

**54.** Vorrichtung nach einem der Ansprüche 48 bis 53, worin die Vorrichtung außerdem Einrichtungen zum Abdecken des Reaktionsprodukts oder der aufsteigenden Schaum-Bahn mit einer Abdeck-Bahn (8) unter Bildung eines Verbundmaterials umfaßt.

**55.** Vorrichtung nach Anspruch 54, worin die Einrichtung zum Abdecken des Reaktionsprodukts oder der aufsteigenden Schaum-Bahn eine Walze (9) einschließt.

**56.** Vorrichtung nach einem der Ansprüche 48 bis 55, welche außerdem Einrichtungen (15) zum Trocknen der porösen Zellschaum-Bahn auf Polymerbasis einschließt, nachdem die Zellschaum-Bahn endgehärtet wurde.

**57.** Vorrichtung nach Anspruch 56, worin die Trockeneinrichtung (15) ein Teilchenfilter umfaßt, so daß die poröse Zellschaum-Bahn auf Polymerbasis im wesentlichen Teilchen-frei bleibt.

**58.** Vorrichtung nach einem der Ansprüche 48 bis 57, worin die Vorrichtung außerdem eine Einrichtung umfaßt, um das Reaktionsprodukt und die aufsteigende Schaum-Bahn im wesentlichen Teilchen-frei zu halten.

**Revendications**

**1.** Procédé continu pour la préparation d'une feuille de mousse à base de polymère dans lequel la mousse est produite à partir d'un produit de réaction durcissable à température ambiante formé par la réaction d'une composition réagissante comprenant un prépolymère et de l'eau, la préparation de ladite feuille de mousse comprenant les étapes consistant à fournir un substrat se déplaçant continûment (5) et à durcir la feuille de mousse à base de polymère, ledit procédé étant caractérisé en ce que la feuille de mousse à base de polymère produite par ledit procédé est une feuille de mousse alvéolaire poreuse d'une épaisseur prédéterminée, le produit de réaction étant déposé sur le substrat (5) à une vitesse et avec une quantité telle que l'épaisseur de la feuille formée par le produit de réaction, si on la laisse s'élever jusqu'à son niveau maximal sans la soumettre à une compression, soit supérieure à ladite épaisseur prédéterminée, à laisser ledit produit de réaction commencer à s'élever pour former une feuille de mousse qui s'élève, à passer ladite feuille de mousse qui s'élève dans et à travers une zone de compression, à comprimer ladite feuille de mousse qui s'élève à un taux prédéterminé, à retirer la feuille de mousse qui s'élève de la zone de compression, à laisser s'élever la feuille de mousse qui s'élève sortant de ladite zone de compression afin de fournir une feuille de mousse alvéolaire poreuse d'épaisseur prédéterminée avant de finalement durcir ladite feuille de mousse alvéolaire poreuse.

**2.** Procédé selon la revendication 1, dans lequel l'épaisseur de la feuille de mousse qui s'élève est maintenue constante pendant la compression de la feuille de mousse qui s'élève dans la zone de compression.

**3.** Procédé selon la revendication 1 ou la revendication 2, dans lequel ledit prépolymère est un prépolymère de polyuréthane.

**4.** Procédé selon la revendication 3, dans lequel ledit prépolymère de polyuréthane est un prépolymère de polyéther contenant des groupes isocyanates.

**5.** Procédé selon l'une quelconque des revendications précédentes, dans lequel ledit substrat (5) adhère de manière détachable audit produit de réaction, à ladite feuille de mousse qui s'élève et à ladite feuille de mousse alvéolaire poreuse.

6. Procédé selon l'une quelconque des revendications précédentes, dans lequel ledit substrat (5) est choisi dans le groupe constitué de poly(chlorure de vinylidène) et de polyuréthane.

7. Procédé selon l'une quelconque des revendications précédentes, consistant en outre à couvrir ledit produit de réaction ou ladite feuille de mousse qui s'élève avec une feuille de couverture (8) après avoir déposé ledit produit de réaction sur ledit substrat (5).

8. Procédé selon la revendication 7, dans lequel ladite feuille de couverture (8) adhère de manière détachable audit produit de réaction et à ladite feuille de mousse qui s'élève.

9. Procédé selon la revendication 7 ou la revendication 8, dans lequel ladite feuille de couverture (8) est constituée soit de papier, soit de matières plastiques.

10. Procédé selon la revendication 9, dans lequel ladite feuille de couverture (8) est constituée de polystyrène.

11. Procédé selon l'une quelconque des revendications 7 à 10, consistant en outre à retirer ladite feuille de couverture (8) après que ladite feuille de mousse alvéolaire poreuse est finalement durcie.

12. Procédé selon l'une quelconque des revendications précédentes, consistant en outre à sécher ladite feuille de mousse alvéolaire poreuse après que ladite feuille de mousse alvéolaire poreuse est finalement durcie.

13. Procédé selon la revendication 12, dans lequel ledit séchage est réalisé par injection d'air sec.

14. Procédé selon la revendication 12 ou la revendication 13, dans lequel ledit séchage est réalisé jusqu'à ce que ladite feuille de mousse alvéolaire poreuse présente une teneur en humidité d'environ 10 % en poids.

15. Procédé selon l'une quelconque des revendications précédentes, dans lequel ledit procédé est réalisé dans un environnement exempt de matières particulaires.

16. Procédé selon l'une quelconque des revendications précédentes, dans lequel ledit taux prédéterminé de compression se trouve dans l'intervalle d'environ 5 % à environ 95 % de l'épaisseur de la feuille de mousse qui s'élève juste avant ladite compression, comme elle est mesurée après qu'on laisse ledit produit de feuille de mousse qui s'élève s'élever jusqu'à son niveau maximal.

17. Procédé selon l'une quelconque des revendications précédentes, dans lequel ladite composition réagissante comprend en outre un agent hydrophile capable d'absorber l'eau.

18. Procédé selon l'une quelconque des revendications précédentes, dans lequel ladite composition réagissante comprend en outre un adjuvant dans la forme d'un alcool.

19. Procédé selon la revendication 18, dans lequel ledit alcool est choisi dans le groupe constitué de monols, de diols et d'alcools polyhydriques.

20. Procédé selon l'une quelconque des revendications précédentes, dans lequel ladite composition réagissante comprend en outre un agent mouillant.

21. Procédé selon l'une quelconque des revendications précédentes, dans lequel ledit prépolymère est choisi dans le groupe constitué de polyols de polyéthers contenant des groupes isocyanates présentant un poids d'équivalent en isocyanate d'environ 0,5 meq/g à environ 2,5 meq/g et de mélanges de ceux-ci.

22. Procédé selon l'une quelconque des revendications précédentes, dans lequel ledit prépolymère est présent dans une quantité d'environ 20 % en poids à environ 50 % en poids de la composition réagissante totale.

**23.** Procédé selon la revendication 17 ou l'une quelconque des revendications 18 à 22 lorsqu'elle dépend de la revendication 17, dans lequel ledit agent hydrophile est un polymère absorbant capable d'absorber l'eau et présentant une absorption de fluide supérieure à environ 50 ml d'eau par gramme dudit polymère.

**24.** Procédé selon la revendication 17 ou l'une quelconque des revendications 18 à 23 lorsqu'elle dépend de la revendication 17, dans lequel ledit agent hydrophile est un élément choisi dans le groupe constitué de copolymères d'acrylates greffés par de l'amidon, de copolymères de sels d'acrylamides et de polyacrylates greffés par de l'amidon et de mélanges de ceux-ci.

**25.** Procédé selon la revendication 17 ou l'une quelconque des revendications 18 à 24 lorsqu'elle dépend de la revendication 17, dans lequel ledit agent hydrophile est présent dans une quantité suffisante pour fournir une composition de mousse capable d'absorber au moins environ 3 fois son poids de liquide.

**26.** Procédé selon la revendication 25, dans lequel ladite composition de mousse est capable de transporter de manière étanche au moins environ 3 fois son poids de liquide.

**27.** Procédé selon la revendication 19 ou l'une quelconque des revendications 20 à 26 lorsqu'elle dépend de la revendication 19, dans lequel ledit alcool est soluble dans l'eau.

**28.** Procédé selon la revendication 19 ou l'une quelconque des revendications 20 à 27 lorsqu'elle dépend de la revendication 19, dans lequel ledit alcool polyhydrique présente un poids moléculaire inférieur à environ 1 000.

**29.** Procédé selon la revendication 19 ou l'une quelconque des revendications 20 à 28 lorsqu'elle dépend de la revendication 19, dans lequel ledit alcool est un élément choisi dans le groupe constitué d'alcool isopropylique, d'éthanol, de propylèneglycol, de polyéthylèneglycol, de polypropylèneglycol, de glycérine, de 1,2,4-butanetriol, de triméthylolpropane, de sorbitol, de pentaérythritol et des mélanges de ceux-ci.

**30.** Procédé selon la revendication 18 ou l'une quelconque des revendications 19 à 29 lorsqu'elle dépend de la revendication 18, dans lequel ledit alcool est présent dans une quantité d'environ 5 % en poids à environ 30 % en poids de la composition réagissante.

**31.** Procédé selon la revendication 20 ou l'une quelconque des revendications 21 à 30 lorsqu'elle dépend de la revendication 20, dans lequel ledit agent mouillant est un tensioactif non ionique choisi dans le groupe constitué de copolymères blocs d'oxyde d'éthylène et d'oxyde de propylène, d'ester d'acides gras de sorbitane éthoxylé, d'esters de glycérol, de poly(esters de glycérol), de fluides de silicone et de mélanges de ceux-ci.

**32.** Procédé selon la revendication 20 ou l'une quelconque des revendications 21 à 31 lorsqu'elle dépend de la revendication 20, dans lequel ledit agent mouillant est présent dans une quantité d'environ 1 % en poids à environ 10 % en poids de ladite composition réagissante.

**33.** Procédé selon la revendication 18 lorsqu'elle dépend de la revendication 17, dans lequel ledit prépolymère, ledit agent hydrophile et ledit adjuvant sont présents dans la composition réagissante de sorte que le rapport du prépolymère à l'agent hydrophile se trouve dans l'intervalle d'environ 20:1 à environ 20:10 et le rapport du prépolymère à l'adjuvant se trouve dans l'intervalle d'environ 20:2 à environ 20:30.

**34.** Procédé selon la revendication 33, dans lequel ledit prépolymère est un élément choisi dans le groupe constitué de polyols de polyéthers contenant des groupes isocyanates présentant un poids d'équivalent en isocyanate d'environ 0,5 meq/g à environ 2,5 meq/g et de mélanges de ceux-ci ; ledit agent hydrophile est un élément choisi dans le groupe constitué de copolymères d'acrylates greffés par de l'amidon, de copolymères de sels d'acrylamides et de polyacrylates greffés par de l'amidon, et de mélanges de ceux-ci ; ledit adjuvant est un élément choisi dans le groupe constitué d'éthanol, d'alcool isopropylique, de propylèneglycol, de polyéthylèneglycol, de polypropylèneglycol, de glycérine, de 1,2,4-butanetriol, de triméthylolpropane, de sorbitol, de pentaérythritol et de mélanges de ceux-ci ;

ladite composition réagissante comprend un agent mouillant et ledit agent mouillant est un élément choisi dans le groupe constitué de copolymères blocs d'oxyde d'éthylène et d'oxyde de propylène, d'esters d'acides gras de sorbitane éthoxylé, d'esters de glycérol, de poly(esters de glycérol), de fluides de silicone et de mélanges de ceux-ci et ladite eau est un élément choisi dans le groupe constitué d'eau désionisée, d'eau distillée et de solutions salines normales.

35. Procédé selon la revendication 34, dans lequel ledit prépolymère est un prépolymère de polyéther contenant des groupes isocyanates présentant un poids d'équivalent en isocyanate d'environ 1,6 meq/g et un poids d'équivalent par groupe isocyanate d'environ 625 ; ledit agent hydrophile est copolymère (2-propénamide, 2-acrylate mixte de sodium et d'aluminium) greffé par de l'amidon ; ledit adjuvant comprend de la glycérine et ledit agent mouillant est un élément choisi dans le groupe constitué de copolymères blocs d'oxyde d'éthylène et d'oxyde de propylène.

36. Procédé selon la revendication 4, lorsque la revendication 4 dépend de la revendication 1, dans lequel le substrat se déplaçant continûment (5) se déplace à une vitesse dans l'intervalle d'environ 0,5 à environ 55,9 mm/s (environ 0,1 à environ 11 pieds/min) et le produit de réaction est déposé sur le substrat (5) à une vitesse dans l'intervalle d'environ 0,8 à environ 15,1 g/s (environ 0,1 à environ 2,0 livres/min).

37. Procédé selon la revendication 36, dans lequel ledit substrat se déplaçant continûment (5) se déplace à une vitesse d'environ 25,4 mm/s (5 pieds/min) et ledit produit de réaction est déposé sur ledit substrat (5) à une vitesse d'environ 1,5 g/s (0,2 livres/min).

38. Procédé selon la revendication 36 ou la revendication 37, dans lequel la température dudit produit de réaction lors de la déposition est comprise entre environ 15,6°C (60°F) et environ 48,9°C (120°F).

39. Procédé selon l'une quelconque des revendications 36 à 38, dans lequel ledit taux prédéterminé de compression se trouve dans l'intervalle d'environ 5 % à environ 95 % de l'épaisseur de la feuille de mousse qui s'élève juste avant ladite compression comme elle est mesurée après qu'on laisse ladite feuille de mousse qui s'élève s'élever jusqu'à son niveau maximal.

40. Procédé selon l'une quelconque des revendications 36 à 39, dans lequel ladite étape de compression consiste à soumettre ladite feuille de mousse qui s'élève à 3 compressions.

41. Procédé selon la revendication 40, dans lequel le taux de la première desdites trois compressions est d'environ 80 % de l'épaisseur de la feuille de mousse qui s'élève juste avant la première compression comme elle est mesurée après qu'on laisse ladite feuille s'élever jusqu'à son niveau maximal, le taux de la seconde compression est d'environ 40 % de l'épaisseur de la feuille de mousse qui s'élève avant la seconde compression comme elle est mesurée après qu'on laisse ladite feuille s'élever jusqu'à son niveau maximal et le taux de la troisième compression est d'environ 40 % de l'épaisseur de la feuille de mousse qui s'élève avant ladite troisième compression comme elle est mesurée après qu'on laisse ladite feuille s'élever jusqu'à son niveau maximal.

42. Procédé selon l'une quelconque des revendications 36 à 41, consistant en outre à couvrir ledit produit de réaction ou ladite feuille de mousse qui s'élève avec une feuille de couverture (8) après avoir déposé ledit produit de réaction sur ledit substrat (5).

43. Procédé selon l'une quelconque des revendications 36 à 42, dans lequel ledit substrat (5) est déplacé par une bande transporteuse (6) se déplaçant continûment.

44. Procédé selon l'une quelconque des revendications 36 à 42, dans lequel ledit substrat (5) est tiré par un dispositif d'enroulement.

45. Procédé selon la revendication 42 ou l'une quelconque des revendications 43 ou 44 lorsqu'elle dépend de la revendication 42, consistant en outre à retirer ladite feuille de couverture (8) après que ladite feuille de mousse qui s'élève est finalement comprimée et que ladite feuille de mousse qui s'élève n'adhère plus à ladite feuille de couverture (8).

**46.** Procédé selon l'une quelconque des revendications 36 à 45, consistant en outre à sécher ledit produit après que ledit produit est finalement durci à une température comprise entre environ 37,8°C (100°F) et environ 79,4°C (175°F).

**47.** Procédé selon la revendication 46, dans lequel ladite étape de séchage est réalisée dans un environnement exempt de matières particulaires.

**48.** Appareil pour la préparation continue d'une feuille de mousse à base de polymère pendant laquelle la mousse est produite à partir d'un produit de réaction durcissable à température ambiante formé par la réaction d'une composition réagissante comprenant un prépolymère et de l'eau, l'appareil comprenant un moyen (1, 2, 3, 4) pour faire réagir ladite composition réagissante et un moyen (6) pour fournir un substrat se déplaçant continûment (5), l'appareil étant caractérisé en ce que ladite feuille de mousse est une feuille de mousse alvéolaire poreuse d'épaisseur prédéterminée, l'appareil comprenant en outre un moyen (7) approprié pour déposer ledit produit de réaction sur le substrat (5) à une vitesse et dans une quantité telle que l'épaisseur de la feuille formée par le produit de réaction, si on la laisse s'élever jusqu'à son niveau maximal sans la soumettre à une compression, soit supérieure à ladite épaisseur prédéterminée et un moyen de compression (9, 10, 11, 12, 13, 14) pour comprimer à un taux prédéterminé une feuille de mousse qui s'élève formée à partir dudit produit de réaction, le substrat (5) acheminant le produit de réaction vers ledit moyen de compression de sorte que le produit de réaction commence à s'élever sur ledit substrat formant ladite feuille de mousse qui s'élève avant de rencontrer le moyen de compression, l'appareil étant tel qu'à la sortie du moyen de compression on laisse la feuille de mousse qui s'élève s'élever pour fournir une feuille de mousse alvéolaire poreuse avant son durcissement final, ledit produit de réaction, ladite feuille de mousse qui s'élève et ladite feuille de mousse alvéolaire poreuse étant réalisées à température ambiante après avoir été déposées sur ledit substrat (5) au moins jusqu'à ce que ladite feuille de mousse alvéolaire poreuse à base de polymère soit finalement durcie.

**49.** Appareil selon la revendication 48, dans lequel le moyen de compression (9, 10, 11, 12, 13, 14) est tel que l'épaisseur de la feuille de mousse qui s'élève est maintenue constante pendant la compression de la feuille de mousse qui s'élève par ledit moyen de compression.

**50.** Appareil selon la revendication 48 ou la revendication 49, dans lequel ledit moyen pour fournir un substrat se déplaçant continûment comprend une bande transporteuse (6).

**51.** Appareil selon la revendication 48 ou la revendication 49, dans lequel ledit moyen pour fournir un substrat se déplaçant continûment comprend un dispositif d'enroulement.

**52.** Appareil selon l'une quelconque des revendications 48 à 51, dans lequel ledit moyen pour déposer ledit produit de réaction comprend un injecteur (7).

**53.** Appareil selon l'une quelconque des revendications 48 à 52, dans lequel ledit moyen pour comprimer ladite feuille de mousse qui s'élève comprend au moins une paire de rouleaux-presseurs (9, 10, 11, 12, 13, 14).

**54.** Appareil selon l'une quelconque des revendications 48 à 53, dans lequel ledit appareil comprend en outre un moyen pour couvrir ledit produit de réaction ou ladite feuille de mousse qui s'élève avec une feuille de couverture (8) pour former un composite.

**55.** Appareil selon la revendication 54, dans lequel ledit moyen pour couvrir ledit produit de réaction ou ladite feuille de mousse qui s'élève comprend un rouleau-presseur (9).

**56.** Appareil selon l'une quelconque des revendications 48 à 55, comprenant en outre un moyen (15) pour sécher ladite feuille de mousse alvéolaire poreuse à base de polymère après que ladite feuille de mousse alvéolaire est finalement durcie.

**57.** Appareil selon la revendication 56, dans lequel ledit moyen de séchage (15) comprend un filtre particulaire de sorte que ladite feuille de mousse alvéolaire poreuse à base de polymère reste pratiquement exempte de matières particulaires.

23

**58.** Appareil selon l'une quelconque des revendications 48 à 57, dans lequel ledit appareil comprend en outre un moyen pour maintenir ledit produit de réaction et ladite feuille de mousse qui s'élève pratiquement exempt de matières particulaires.